# EUROPEAN PATENT APPLICATION

(11) **EP 4 190 367 A1**
(43) Date of publication of application: **07.06.2023**
(21) Application number: 21853023.6
(22) Date of filing: 01.04.2021
(51) Int. Cl.: A61L 2/10, H05B 47/00

(54) **DEVICE FOR PHOTOINACTIVATION OF PATHOGENIC BIOLOGICAL AGENTS**

(30) Priority: 03.08.2020 RU 2020125752
(71) Applicant: Macuk, Igor Vladislavovich, Moscow, 125080 (RU)
(72) Inventor: KUZ'MIN, Oleg Viktorovich, Krasnodar, 350018 (RU); FASKHUTDINOVA, Nadezhda Il'gizarovna, Krasnodar, 350018 (RU)
(74) Representative: Perronace, Andrea
(86) International application number: PCT/RU2021/050088
(87) International publication number: WO 2022/031189

(57) **Abstract**

The invention relates to devices for decontaminating air and surfaces in enclosed spaces in the presence and in the absence of people. A lighting device comprises: a device for the photoinactivation of pathogenic biological agents which includes: a first group of sources of radiation, consisting of sources that generate white light having a colour temperature value of from 3000 K to 6000 K and an opening angle Θ_{0.9V} of from 10 to 180 degrees, and sources that generate ultraviolet light in a range of 300-400 nm with an opening angle Θ_{0.9V} of from 10 to 180 degrees; and a second group of sources of radiation that generate ultraviolet light in a range of 300-400 nm and/or 200-300 nm with an opening angle Θ_{0.9V} of from 10 to 180 degrees. In the presence of people, the set irradiance of the sources of ultraviolet radiation at a distance of two metres from the floor of the enclosed space is not greater than the permissible safe radiant exposure, and in the absence of people, the set irradiance of the sources of ultraviolet radiation is not subject to limitations and is at least equal to the radiant exposure required for the photoinactivation of pathogenic biological agents of risk groups 1-4 within the shortest possible time.

## Description

The invention refers to the field of lighting engineering and can be used for disinfection and artificial lighting of any premises, including those of health care facilities, pharmaceutical and food industry enterprises, airports, train stations, metro stations, and other mass gathering sites.

### Technical Field

The invention relates to LED devices for disinfecting the air and surfaces with or without the presence of people in the premises.

### Prior Art

In the wavelength range of 200-450 nm, the light is known to have bactericidal properties /Downes, A., Blunt, T. The Influence of Light upon the Development of Bacteria 1. Nature (1877), 16, 218, https://doi.org/10.1038/016218a0/.

This range can be classified by the following spectrum areas: Ultraviolet (UV) radiation (UVC of 200-280 nm, UVB of 280-315 nm, UVA of 315-400 nm) and visible radiation of 400-450 nm.

In its UVC range (with the maximum impact at 265 nm), UV radiation is the most effective for inactivating pathogenic biological agents, as it is well absorbed by RNA and DNA molecules contained in viruses, bacteria and cells of all living organisms. Photochemical destructive reactions lead to the formation of covalent RNA-protein bonds, as well as to the formation of a covalent bond between two adjacent pyrimidine DNA bases.

It is categorically forbidden to use UVC radiation in the presence of humans and animals, since it is a major factor associated with the induction and progression of skin cancers, retinal burns, and visual impairment. Therefore, the disinfection by using UVC radiation is carried out only periodically and when there are no people in the premises. Such disinfection is ineffective because, when people come back to the premises, the bacterial and viral contamination restarts to grow. A safer wavelength range of UVB, UVA and short-wavelength of visible light may provide an alternative to the use of UVC radiation and enable continuous disinfection in the presence of humans and animals.

The combination of bactericidal radiation source with artificial light source in a single device enables the implementation of a lighting device with disinfecting function.

A known patent US2291926A "Source of visual and ultra violet radiation" published on August 4, 1942 refers to the use of ultraviolet light sources in combination with visual lighting sources.

A known patent US2339010A "Combined light fixture and sterile lamp" published on January 11, 1944, refers to the light fixture designed for the use in sterile or antiseptic conditions within the food industry.

A known patent US2434980A "Combination illuminating and sterilizing lamp" published on January 27, 1948, refers to combined illuminating and sterilizing elements.

In terms of the state of the art, the main disadvantage of these technical solutions is the use of environmentally hazardous mercury lamps for inactivating microorganisms and fluorescent lamps for general lighting, as well as the lack of control devices for lighting and disinfection processes.

Semiconductor light sources, such as light-emitting diodes (LEDs) and laser diodes that emit in the ultraviolet and short-wave visible spectrum, became commercially available only in the first decade of the 21st century. The benefits of semiconductor light sources are obvious, as they are highly efficient and environmentally friendly with no content of mercury or other components hazardous for humans and the environment. In addition, given the compact size of SMD LEDs, their radiation can be combined with different wavelengths to optimize the inactivation of pathogenic biological agents.

A known utility model patent RU101635U1 "Indoor air and surface irradiation unit" published on January 27, 2011, refers to indoor air and surface disinfection devices. Such device is designed as a matrix with LEDs of white light, infrared light of 850 nm, and ultraviolet light of 390 nm and 265 nm. It operates as follows. LEDs with a wavelength of 265 nm are activated for 10-20 minutes when there are no people in the premises. When people arrive to the premises, LEDs of white light are activated for illumination and LEDs with a wavelength of 390 nm are activated to increase the immunity in those present in the premises. LEDs with a wavelength of 850 nm are activated as needed to heat the working area.

The disadvantage of the known device is its inability to provide effective disinfection of the air and surfaces for a long time.

With sufficient irradiance, the radiation at the wavelength of 265 nm can inactivate viruses and bacteria in 15-20 minutes. With people coming back to the premises, the uncontrolled contamination of the air and surfaces with biological pathogenic agents will resume. At 390 nm, the radiation has a bactericidal effect only on the bacteria that produce exogenous chromophores absorbing such radiation. As for the viruses, the radiation at the wavelength of 390 nm cannot destroy them.

A known patent US8398264B2 "Lighting device" published on March 19, 2013, refers to lighting devices for inactivating medically relevant bacteria. This device uses monochromatic LED radiation in the wavelength range of 380-420 nm to inactivate the bacteria and LEDs that emit visible light at a different wavelength, with the total output signal of the device generating white light. LEDs in the wavelength range of 380-420 nm and LEDs emitting white light can operate together or separately. LEDs providing white light and LEDs for inactivating the bacteria operate jointly in the presence of humans. When there are no people in the premises, only LEDs in the wavelength range of 380-420 nm operate and their irradiance increases.

The device according to that invention can inactivate only the bacteria and has a significant disadvantage.

In the lighting device, the group of LEDs in the wavelength range of 380-420 nm operates longer than the group of LEDs providing the white light. Therefore, the group of LEDs in the wavelength range of 380-420 nm will deplete its resources earlier than the group of LEDs emitting white light, and the device will be able to continue operating only as an artificial light source for the premises.

A known patent US9333274B2 "Disinfecting light fixture" published on May 10, 2016 refers to lighting devices that provide high-quality lighting along with disinfection of the environment by using the light in the wavelength range of 380-420 nm which can reduce or eliminate bacterial, fungal and/or viral contamination on the surfaces of the environment.

The light fixture contains the first light source, that emits at a peak wavelength ranging from 380 nm and 420 nm, and the second light source, that emits at a different wavelength, and such second light source may contain the third and fourth light sources, while the combined luminous flux is perceived as the white light. The disadvantage of this technical solution is the low efficiency of bacteria inactivation and the lack of the claimed ability to eliminate the viral contamination on the surfaces of the environment.

This light fixture operates only in the lighting mode with a simultaneous disinfection function using the white light. With the illumination at 500 lux, the efficiency of bacteria inactivation stands at 99.7% after 14 hours of operation.

It is known that the lighting is considered to be normal if a person can stay in the premises for a long time and maintain the ability to work without noticing any unreasonable deterioration in health. When the lighting becomes too intense, the person has a feeling of general discomfort, dry eyes, blurry vision and exhibits hyperarousal. In hospital setting, the calm and comfort of patients is achieved at 150-250 lux, the lighting of kindergarten playrooms should not exceed 200 lux; for classrooms, 300 lux; for offices, 300 lux; for gyms, 200 lux.

The bacteria are cumulative photobiological receptors; therefore, if at 500 lux the bactericidal contamination is reduced by 99.7% after 14 hours of operation, then achieving a similar effect in hospital wards at the recommended illumination of 250 lux would require to operate such light fixture continuously for at least 28 hours. The radiation in the wavelength range of 380-420 nm is used to inactivate the bacteria containing endogenous chromophores that absorb such radiation. The viruses contain no chromophores that can absorb radiation in the wavelength range of 380-420 nm, so they cannot be inactivated at low power density of such radiation.

A known patent US10363325B2 "Lighting device that deactivates dangerous pathogens while providing visually appealing light" published on June 30, 2019, refers to lighting devices designed to provide light and disinfect air in their environment.

The lighting device contains a housing, a fixture for attaching the housing to a surface, one or more first LEDs to produce disinfecting light in the first wavelength range, and one or more second LEDs to produce disinfecting light in a second wavelength range different from the first wavelength range.

The disinfecting lights generated by the first LEDs and the second LEDs are mixed to form a combined visible light other than white light with correlated color temperatures ranging between 1500K and 7000K.

Such lighting device may include an HVAC unit or device to heat the air near the lighting device to a temperature higher than the air temperature, thereby creating an air convection current proximate to the lighting device.

The lighting device may additionally contain one or more third LEDs arranged in the housing and configured to each produce disinfecting light having a wavelength in a third wavelength range different from the first and second wavelength ranges. LEDs generate disinfecting light in the first wavelength range of 400-420 nm, in the second wavelength range of 530-580 nm and in the third wavelength range of 600-650 nm.

This technical solution has a number of disadvantages.

The photoinactivation of bacteria will occur only under the impact of LED radiation in the wavelength range of 400-420 nm, because such radiation is absorbed by porphyrins, which act as endogenous photosensitizers of bacteria.

The contribution of the second wavelength range of 530-500 nm to photoinactivation of bacteria will be negligible, and such contribution of the third wavelength range of 600-650 nm is doubtful.

The mix of violet light of 400-420 nm with green-yellow light of 530-550 nm or orange-red light of 600-650 nm will create a light different from the white light, which will not meet the requirements for artificial lighting sources in schools, healthcare facilities, or workplaces at enterprises.

It would not be practical to add an HVAC unit or heating element to the lighting fixture, unless it is stipulated by the technical requirements for the premises because, during its operation, any LED device emits heat, since only 40% of the electrical power consumed by LEDs is converted into light, while 60% is converted into heat which creates an air current near the lighting device to remove the heat from the device to the environment.

A known patent US10363327B2 "Luminaire with white light LEDs and UV LEDs for lighting and disinfection" published on July 30, 2019, refers to various applications of UV LEDs for disinfecting air, water, and surfaces.

The combined luminaire for disinfection and lighting contains a first set of LEDs, a second set of LEDs, and a third set of LEDs that are housed in one luminaire.

The first set of LEDs is designed to produce white light to illuminate a room when energized.

The second set of LEDs generating invisible light in the UV range is directed downward to disinfect surfaces in the room when energized.

The third set of LEDs generating invisible light in the UV range and its UV light is not directed downward and is intended for disinfecting air in the room when energized.

The luminaire contains a sensor for detecting whether there are people in the vicinity of the luminaire, and the sensor is connected to automatic control system of the second set of LEDs.

The luminaire is configured to operate in at least two modes.

The luminaire operates in the first mode, when the sensor detects a person in the vicinity of the luminaire, and the second set of LEDs automatically ceases to emit the downward light, while the third set of LEDs remains energized to disinfect the air.

The luminaire operates in the second mode when the sensor detects no people in the vicinity of the luminaire, and then the second set of LEDs and the third set of LEDs are energized to disinfect surfaces in the room and disinfect air in the room.

A significant disadvantage of this technical solution is its inability to effectively disinfect the air and surfaces.

According to the invention's description, when people are present in the room, only the set of white light LEDs and the set of UV LEDs for air disinfection, the radiation of which is not directed downwards, are operated. When people sneeze or cough, they tend to lower their heads or cover their mouth and nose with their hands. Therefore, the aerosol droplets will not go upwards to the UV source, but will settle on the surrounding surfaces under the action of gravity.

According to the claims and description of the invention, the surfaces are disinfected by a set of UV LEDs, which are energized when there are no people in the room or in the vicinity of the radiation source.

In fact, this means that the luminaire cannot effectively disinfect the air and surfaces in the premises where people are present all the time, such as in the wards for patients with burns, surgery or in infection departments, airports, railway and sea stations, etc. After a certain period of time, the set of LEDs for disinfection of air will fail first, and the set of LEDs for general lighting and the set of LEDs for disinfection of surfaces in the room without people will continue to operate.

### Summary of Invention

The availability of means to inactivate viruses and bacteria safely for humans will significantly prevent the global spread of infectious diseases. The purpose of this invention is to create a manufacturable lighting device that provides general lighting and effective photoinactivation of pathogenic biological agents in the maximum possible area of the premises without any adverse or hazardous implications for the people present in such premises.

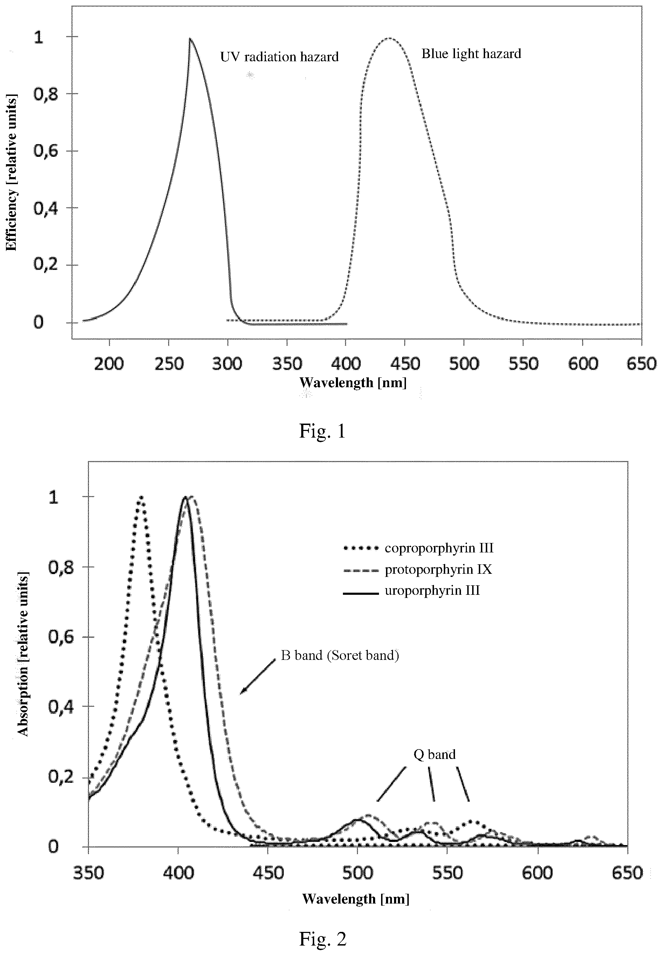

According to ICNIRP (GOST R IEC 62471-2013), the light in the wavelength range of 300-400 nm represents the minimum light-related biological hazard to the human retina (Fig. 1).

The primary photochemical processes resulting in the death of viruses and bacteria occur in the wavelength range of 300-310 nm, as this range of light causes damage to the capsid proteins of viruses and the cytoplasmic membrane proteins of bacteria. The metabolic products of many bacteria are porphyrins, such as coproporphyrin III, protoporphyrin IX, and uroporphyrin III. Porphyrins have extremely high molar coefficients of light absorption and can enter into photochemical oxidative reactions resulting in the formation of reactive oxygen species and free radicals, which irreversibly destroy the cellular structures in bacteria.

The absorption spectra of porphyrins are determined by the chemical structure of molecules and include a strong B band (Soret band) in the 350-420 nm range and dozens of times less intense Q band in the 500-600 nm range (Fig. 2).

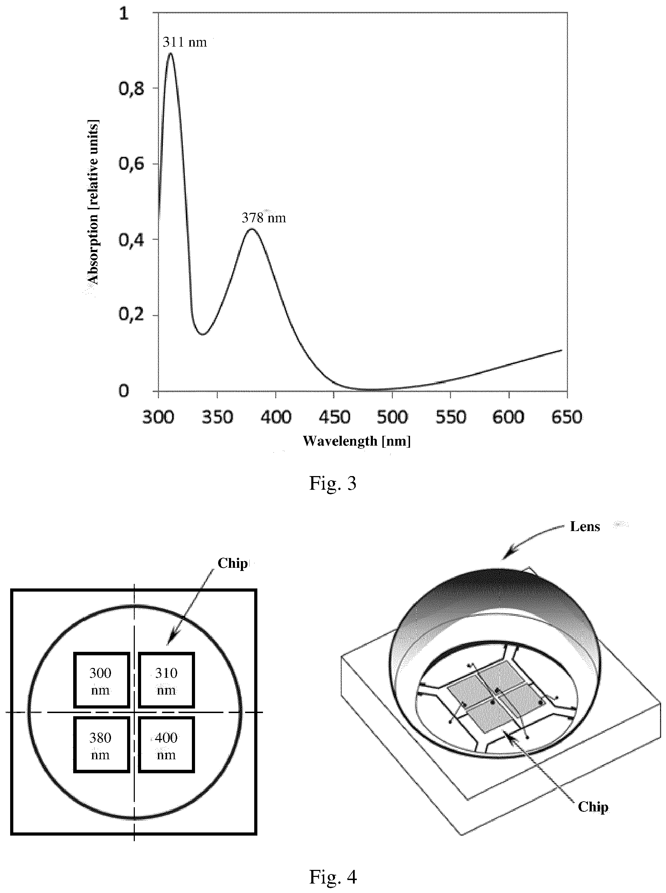

The authors of the invention found that, along with porphyrins, the exogenous photosensitizers may include natural bacterial pigments, such as pyocyanin, which is a specific metabolite of *Pseudomonas aeruginosa* with the absorption spectrum that has a band with a peak absorption at 311 nm and 378 nm (Fig. 3), therefore, the use of several radiation sources with different wavelengths will increase the effect of inactivating the pathogenic bacteria /N.I. Faskhutdinova, O.V. Kuzmin. Photoinactivation of antibiotic-resistant strains of Pseudomonas aeruginosa. Meditsinskaya Fizika (2017), 4 (76), pp. 37-44 (in Russian). https://elibrary.ru/item.asp?id=32475252/.

Given the above, in the wavelength range of 300-400 nm (which is safe for humans), UV light can be used to inactivate viruses and bacteria. Since this light is practically not perceived by the human eye, its use without white light sources, for example, at night in hospital wards, will not have a negative depressing effect on the nervous system of patients.

As noted earlier, in the range of 200-300 nm, the light is highly effective in inactivating pathogenic biological agents. Therefore, depending on the purpose and requirements for the premises, the light in the wavelength range of 300-400 nm can be used in the presence of people, and the light in the wavelength range of 200-300 nm can be used in the absence of people.

In the wavelength range of 200-300 nm, the exposure to light breaks the chemical bonds of organic compounds, which causes rapid aging of plastics, rubber, and other materials. Given the presence of high-tech equipment and medicines in the premises of health care institutions and pharmaceutical plants, the use of radiation in the range of 300-400 nm would be preferable for disinfection.

### Design features of the device according to the invention

The device contains two groups of LEDs. The first group consists of LEDs emitting white light and LEDs emitting UV light. The LEDs of the first group have a continuous operating life of 50,000 hours. The second group includes LEDs that emit UV light in the wavelength range of 300-400 nm and/or 200-400 nm and also have a continuous operating life of 50,000 hours. Since the first and second groups of LEDs operate alternately (approximately the same number of hours per day), the operating life of the lighting device increases to 100,000 hours.

LEDs emitting UV light can be monochromatic or polychromatic and consist of multiple semiconductor crystals (chips) emitting in the UV spectrum at different wavelengths and integrated into a single housing (Fig. 4). The lenses installed on the LEDs have an opening angle Θ_{0.9V} ranging from 10 degrees to 160 degrees and allow to evenly distribute the intensity of radiation on objects at different distances from the device and increase the effectiveness of disinfection.

The manufacturability of the device is achieved by combining the housing, heatsink and printed circuit board (PCB) in one product. PCB can be manufactured using MCPCB technology, where the heatsink housing serves as the metal base for PCB, thus eliminating the thermal resistance at PCB-heatsink interface and the need for traditional use of heat conducting pastes and adhesives. The additional heat dissipation and sealing of PCB is achieved by filling it with a thermal conductive compound, such as Dow Coming SYLGARD 184 elastomer, up to the level of LED optical elements (lenses).

The base of the heatsink housing can be made in the form of a truncated pyramid or truncated cone, which allows to increase the area of surfaces and air volume disinfected in the premises.

### Brief Description of the Drawings

Fig. 1 shows the spectral function of UV and blue light hazard for the retina according to ICNIRP.
Fig. 2 shows the absorption spectra of porphyrins.
Fig. 3 shows the absorption spectrum of pyocyanin.
Fig. 4 shows an embodiment of polychromatic LED with emission wavelengths of 300 nm, 310 nm, 380 nm, 400 nm.

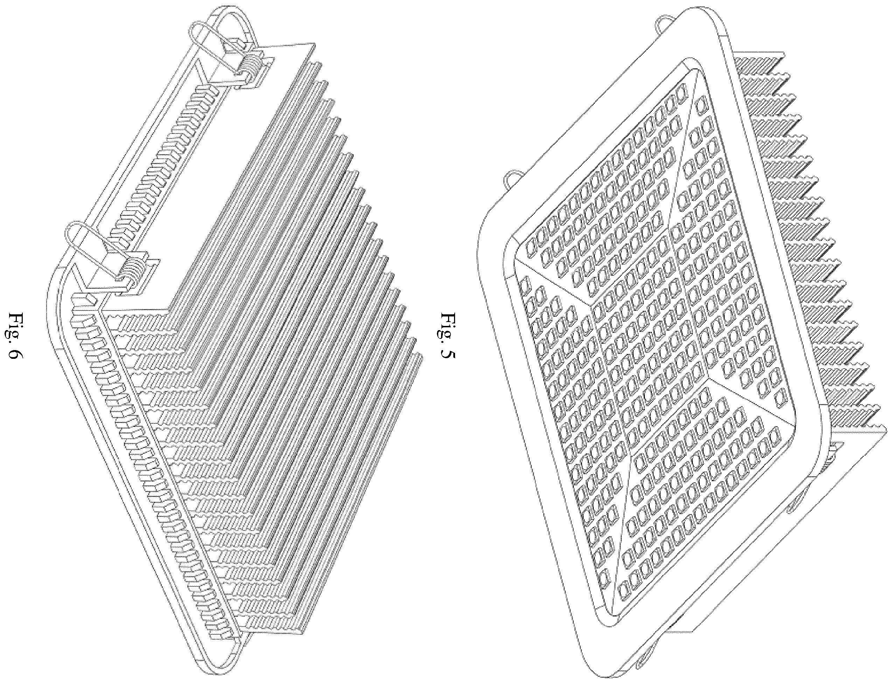

Fig. 5 shows an embodiment of the device, bottom view.
Fig. 6 shows an embodiment of the device, top view.
Fig. 7 shows a variant of arranging LEDs on PCB of the device, where 1a indicates white light LEDs of the first group, 1b indicates UV LEDs of the first group, 2 indicates UV LEDs of the second group.
Fig. 8 shows the main components of a lighting device, where 3 indicates the heatsink housing, along with PCB made on its base and LEDs, 4 indicates the frame for attaching the device to the surface, 5 indicates the elements for attaching the device to the surface for installation.

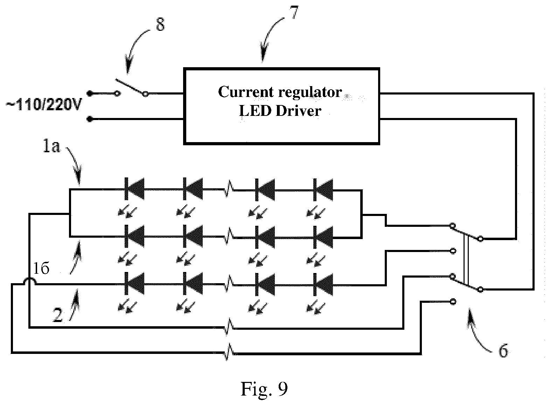

Fig. 9 shows a variant of the diagram for connecting the device to the mains.

### Description of the Preferred Embodiment

The preferred embodiment of the device includes the following:
First group of light sources, which is intended for general lighting and disinfection of air and surfaces in the presence of people and consists of white light SMD LEDs (1a) with a correlated color temperature of 3500K and 4000K and the opening angle of light Θ_{0.9V} of 160 degrees and UV SMD LEDs (1b) emitting simultaneously at wavelengths of 300 nm, 310 nm, 380 nm, and 400 nm with the opening angle of light Θ_{0.9V} of 60 degrees and 120 degrees, and the total spectral irradiance measured at a height of two meters from the floor of premises does not exceed the safe radiant exposure of 10 J/m2, and the light sources of the first group are combined into a single electrical circuit and activated when supplied with power;
Second group of light sources (2), which is intended for disinfecting the air and surfaces in the absence of people in the premises and consists of polychromatic SMD LEDs emitting simultaneously in multiple wavelengths ranging from 200 nm to 300 nm and the opening angle of light Θ_{0.9V} of 60 degrees and 120 degrees, and the established irradiance of UV radiation sources at the surface of the floor in the premises is not limited and corresponds at least to the radiant exposure required for eradicating the biological agents of pathogenicity groups I-IV as soon as possible, and the light sources of the second group are combined into a single electrical circuit and activated when supplied with power;
Heatsink housing (3) made of aluminum alloy with a high thermal conductivity coefficient, and the base of the heatsink housing is shaped as a truncated pyramid with a dihedral angle of 5 degrees at the base, and such base serves as the base for PCB made using MCPCB technology for connecting power circuits to the first and second group of light sources and for installing them;
Frame (4) for attaching the device to aluminum alloy surface;
5 - elements for attaching the device to the surface for installation.

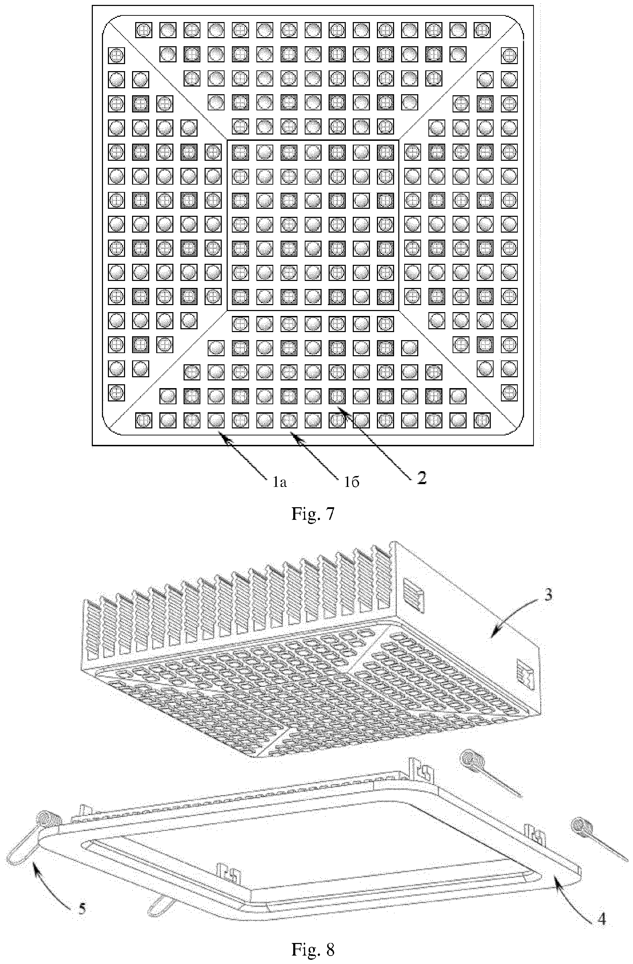

Fig. 8 shows the diagram for connecting the device to the mains, where the first group of LEDs (1a and 1b), as well as the second group of LEDs (2) is connected via the switch (6) to the current regulator (7), which is connected via the breaker (8) to AC mains of 110/220V. The device operates as follows: When the AC mains voltage of 110/220V is supplied to the current regulator (7) via the breaker (8), the switch (6) powers the first group of light sources (1a and 1b) or the second group of light sources (2).

To those skilled in the art it should be clear that the above example of connecting the device to the mains and the example of its manual operation are not the only ones. Modern technology allows to control a device/group of devices using automated disinfection and lighting control systems, including the digital systems, for example, via the DALI protocol compliant with IEC 60929.

## Claims

1. Device for photoinactivation of pathogenic biological agents, comprising:
First group and second group of radiation sources, wherein
First group consists of radiation sources generating white light with a correlated color temperature of 3000K and 6000K and the opening angle Θ_{0.9V} ranging from 10 degrees to 180 degrees and radiation sources generating monochromatic and/or polychromatic UV light in the wavelength range of 300-400 nm with the opening angle Θ_{0.9V} ranging from 10 degrees to 180 degrees, and the total spectral irradiance measured at a height of two meters from the floor of premises does not exceed the safe radiant exposure;
Second group consists of radiation sources generating monochromatic and/or polychromatic UV light in the wavelength range of 300-400 nm and/or 200-300 nm and the opening angle Θ_{0.9V} ranging from 10 degrees to 180 degrees, and in the presence of people, the established irradiance of UV radiation sources at the distance of two meters from the floor of premises does not exceed the allowable safe radiant exposure; and, in the absence of people, the established irradiance of UV radiation sources is not limited and corresponds at least to the radiant exposure required for photoinactivation of biological agents of pathogenicity groups I-IV as soon as possible;
Heatsink housing which is made of material with a high thermal conductivity coefficient and the base of which serves as the base for MCPCB to connect power supply circuits to the first and second group of light sources and install them;
Frame for attaching the device to the surface and elements to attach the device to the surface for installation.

2. Device according to claim 1, **characterized in that** UV radiation sources of the first group are monochromatic and/or polychromatic LEDs, and the sources of the second group are monochromatic and/or polychromatic LEDs and/or lamps.

3. Device according to claim 1 **characterized in that** that heatsink housing has a base shaped as a circle or ellipse, or cone, or truncated cone with an angle between the generatrix and the plane of the cone base greater than 0.5 degrees, a square or rectangle, or polyhedron, or pyramid, or truncated pyramid with a dihedral at the base greater than 1 degree.

4. Device according to claim 1, **characterized in that** heatsink housing, PCB and the frame for attaching the device to the surface are made separately or as a single product.

5. Device according to claim 1, **characterized in that** it includes a fan, temperature sensor, presence and/or motion sensor, light sensor, acoustic sensor, distance sensor, time relay, manual and/or remote controller with devices and detectors to measure radiation characteristics, such as power, energy, irradiance, radiant exposure, radiance.
